# EUROPEAN PATENT APPLICATION

(11) **EP 1 647 494 A2**
(43) Date of publication of application: **19.04.2006**
(21) Application number: 05027149.3
(22) Date of filing: 17.11.2000
(51) Int. Cl.: B65D 65/46, A61K 9/48

(54) **Injection moulded water-soluble container**

(30) Priority: 17.11.1999 GB 9927144; 15.02.2000 GB 0003304; 04.04.2000 GB 0008174; 30.08.2000 GB 0021242
(62) Divisional of application: 04000430.1
(71) Applicant: Reckitt Benckiser (UK) Limited, Slough, Berkshire SL4 3HD (GB)
(72) Inventor: Beckett, Arnold Heyworth, Fox Hill Upper Norwood London, SE19 2UN (GB); Duffield, Paul John, Cherry Burton Beverley, HU17 7RH (GB); Edwards, David Brian, Stevenage Hertfordshire, SG2 7HG (GB); Hammond, Geoffrey Robert, Hull, HU8 0LG (GB); McCarthy, John William, Shaftesbury Dorset, SP7 9DL (GB)
(74) Representative: Bowers, Craig Malcolm

(57) **Abstract**

The present invention provides a rigid, water-soluble container made of an injection moulded poly(vinyl alcohol) and/or a cellulose ether, which container encases a fabric care, surface care or dishwashing composition; and a capsule container comprising at least two components made of one or more material(s) that can be moulded and which are water soluble or water dispersible or in which a substantial part of the surface of these components is water soluble or water dispersible so as to leave perforations throughout the wall when the capsular container is placed in contact with an aqueous environment, wherein the container has one to six compartments, preferably one or two or three, the content of the various compartments being accessible to the aqueous environment when the capsular container is exposed to such an aqueous environment, the accessibility time of the various compartments being the same or different from one compartment to another compartment, with the proviso that the content of the container is not a fabric care, surface care or dishwashing composition.

## Description

The present invention relates to injection-moulded capsule, containers, in particular to such containers that may be utilised for the delivery into an aqueous environment of substances such as detergents, pesticides, biocides, deodorants, dyes and pigments, and water-treatment chemicals.

Clothes washing compositions may be delivered to a clothes washing machine by a delivery tray from which the composition is fed into the washing drum, or they may be placed directly into the washing drum. The washing compositions may be in powder, liquid or block form. Liquid compositions have the disadvantage that they may be spilt. The same applies to powder compositions. Powder compositions have the additional disadvantage that they may produce dust which can be inhaled. These problems are overcome or lessened when blocks of washing composition are used. These are normally individually wrapped. On unwrapping a block, for use, it is still possible that some dust may be produced. Additionally it is an inconvenience for the consumer to have to unwrap the block. Furthermore it is almost impossible for the user to avoid some contact between the block and his or her skin, so leading to a requirement for the user to wash their hands after starting the washing machine. In fact, all of the methods described involve a risk of contact between the composition and the skin, and it is desirable in all cases for the user to wash their hands after starting the washing machine. In this context it should be borne in mind that many compositions contain enzymes to assist the cleaning action. Even though the user may tolerate enzyme residues which may be left in clothes after washing, they may still not tolerate contact between the concentrated washing composition containing the enzymes, and the skin.

Similar considerations apply in relation to other areas including fabric care, surface care and dishwashing. Thus, in relation in particular to dishwashing compositions, there are also problems of spillage, dust generation, skin contact and inconvenience.

It is known to package chemical compositions which may be of a hazardous or irritant nature in water-soluble or water-dispersible materials such as films. The package can simply be added to water in order to dissolve or disperse the contents of the package into the water.

For example, WO 89/12587 discloses a package which comprises an envelope of a water-soluble or water-dispersible material which comprises a flexible wall and a water-soluble or water-dispersible heat seal. The package may contain an organic liquid comprising, for example, a pesticide, fungicide, insecticide or herbicide.

CA-A-1,112,534 discloses a packet made of a water-soluble material in film form enclosing within it a paste-form, automatic dishwasher-compatible detergent composition. The water-soluble material may be, for example, poly(vinyl alcohol), polyethylene oxide or methyl cellulose.

It is also known to form water-soluble containers by thermoforming a water-soluble material. For example, WO 92/17382 discloses a package containing an agrochemical such as a pesticide comprising a first sheet of nonplanar water-soluble or water-dispersible material and a second sheet of water-soluble or water-dispersible material superposed on the first sheet and sealed to it by a continuous closed water-soluble or water-dispersible seal along a continuous region of the superposed sheets.

The above methods of packaging have, however, a number of disadvantages.

The first disadvantage is that they do not have a particularly attractive appearance. In fields such as containers used in the domestic environment, an attractive appearance for an article is extremely desirable. Liquids contained in envelopes of water-soluble film can have a limp, unattractive appearance.

The second disadvantage is that it is difficult to form two or more separate compartments in the packaging so that two incompatible components are both enclosed but separated from each other. Although an arrangement has been described to separate incompatible materials in flexible pouches in WO 93/08095, the method proposed is complex and is not currently achievable in large-scale manufacturing. It cannot, therefore, be used for producing large numbers of containers.

The third disadvantage is that there is only limited control of the release profile of the compositions held in the containers. For example, when a composition is held between two planar water-soluble films or in a thermoformed package, the composition is simply released at the time when the films dissolve or disperse in water. While it may be possible to control to a certain extent the timing of the start of release of the contents, there can be no control over the rate of release of the contents since the entire film dissolves or disperses at about the same time. Furthermore it can be difficult to provide an extended time before the contents of the package are released. An additional problem also arises with thermoformed packages. If the thermoforming is not carefully controlled there may be inadvertent thinning of the film material at the points where the material is drawn down into the mould when it is thermoformed. This could release the contents of the package early. Additionally, in all of the above packages, it is not possible to release different compositions at different times or at different rates since, as discussed above, it is not possible to incorporate more than one composition in each water-soluble container.

The fourth disadvantage is that the containers cannot be produced at a particularly fast rate. When the containers are produced by heat-sealing planar films or by thermoforming, the containers have to be immediately filled and sealed. All of these procedures have to be carried out in succession. This means that it is not possible to obtain a quick throughput for mass-market goods such as household products. For example, standard thermoforming machines can only produce around 400 to 800 containers per minute.

There are numerous forms of systems used in the delivery of medical preparations in the market place today. The two most dominant in relation to oral routes are capsules made from hard gelatine, and tablets - the so-called solid dose formulations. Both of these presentations have remained virtually unchanged for decades. Gelatine capsules are made by a dipping process, building up successive layers, while tablets are formed by compressing a powder or fine granules.

It has now been appreciated that the above type of capsule has utilisations other than in medicine and the human or animal body. In particular, it has been realised that many substances that must be packaged for delivery to their use site could, where that site is an aqueous environment, be contained in similar, though somewhat larger, capsules. Thus, a capsule-like container - a "capsular" container - could be employed to deliver, for example, detergents to a washing machine. Moreover, by appropriately dimensioning the various parts of the container, or by suitably selecting the materials from which they are made, different parts of the container will in use dissolve at different times.

The present invention seeks to provide water-soluble containers which overcome some or all of the above disadvantages.

The present invention has a number of different aspects and embodiments as follows:

The present invention provides an injection-moulded capsule container of any size or shape for the delivery of a water-destined ingredient selected from a fabric care, surface care or dishwashing composition, which container is made of a material that will dissolve in the intended aqueous destination site, said material not being a poly(vinyl alcohol).

The present invention also provides an injection-moulded capsule container of any size or shape for the delivery of a water-destined ingredient selected from a fabric care, dishwashing, water-softening, laundry, rinse aid or antibacterial composition or a refill composition for a trigger-type spray, which container is made of a material that will dissolve in the intended aqueous destination site.

The present invention further provides a method of ware washing, comprising use of a container as defined above, the method entailing introducing the container into a ware washing machine prior to commencement of the washing process, the container being entirely consumed during the washing process. The ware washing machine may, for example, be a dishwashing or laundry washing machine.

The container of the present invention may comprise at least two components made of one or more material(s) that can be moulded and which are water soluble or water dispersible or in which a substantial part of the surface of these components is water soluble or water dispersible so as to leave perforations throughout the wall when the capsular container is placed in contact with an aqueous environment, wherein the container has one to six compartments, preferably one or two or three, the content of the various compartments being accessible to the aqueous environment when the capsular container is exposed to such an aqueous environment, the accessibility time of the various compartments being the same or different from one compartment to another compartment.

The following description and drawings all relate to each and every aspect and embodiment as discussed above, either singly or in any combination thereof.

The containers of the present invention overcome some or all of the above disadvantages.

Firstly, because the containers are rigid and self-supporting, they have an attractive, uniform appearance which does not vary between different containers. Furthermore, the rigid containers can easily have various elements incorporated which are considered to be pleasing to the eye but which are impossible to incorporate in the flexible containers discussed above.

Secondly, because the containers are rigid, it is easily possible to introduce two or more compartments, or have larger compartments separated by walls, to separate mutually incompatible ingredients. The containers can also hold part of the composition on an external surface, for example in an indentation. Furthermore, the container can be moulded is almost any shape that might be useful. In particular it can be given raised or lowered areas.

Thirdly, it is possible to control the release profile of the contents of the container. Since the container is rigid, it is possible to adapt the width of all of the walls of the container to control both the start of release of the composition as well as the rate of release. For example, one or more walls may be made thin in order to have an early release of the composition. Alternatively all the walls may be thick in order to ensure that there is a delayed release of the composition. The rate of release of the composition may also be controlled by ensuring that only part of the container has thin walls which are dissolved or dispersed before the remainder of the container. Different walls or parts of walls of the container may be prepared from different water-soluble polymers which have different dissolution characteristics. For example, a first compartment may be fully enclosed by a polymer such as PVOH which dissolves at a higher or lower temperature than the polymer enclosing a second compartment. Thus different components can be released at different times. If the container holds a solid or gelled composition, it is not even necessary for the container to fully enclose the composition. A part may be left exposed, so that it immediately begins to dissolve when added to water.

Fourthly, since the containers are rigid and self-supporting, they can easily be filled on a production line using normal filling equipment. Such filling equipment is quite capable of filling at least 1500 containers per minute.

Desirably the container, apart from its contents, consists essentially of the injection-moulded polymer. It is possible for suitable additives such as plasticizers and lubricants to be included. Plasticizers are generally used in an amount of up to 20 wt%, for example from 15 to 20 wt%, lubricants are generallly used in an amount of 0.5 to 5% wt% and the polymer is generally therefore used in an amount of 75 to 84.5 wt%, based on the total amount of the moulding composition. Examples of suitable polymers are PVOH and cellulose ethers such as HPMC.

PVOH is a known water-soluble material which is used to prepare water-soluble films for encasing compositions as discussed above. Cellulose ethers have not in general been used to prepare water-soluble films because they have poor mechanical strength.

PVOH materials, unlike gelatin, can be modified to dissolve at different rates under various conditions (including the pH of the aqueous medium into which they are introduced).

The PVOH preferably used to form the container of the present invention may be partially or fully alcoholised or hydrolysed. For example it may be from 40-100%, preferably 70-92 %, more preferably about 88%, alcoholised or hydrolysed polyvinylacetate. The polymer such as PVOH or cellulose ether is generally cold water (20°C) soluble, but may be insoluble in cold water at 20°C and only become soluble in warm water or hot water having a temperature of, for example, 30°C, 40°C, 50°C or even 60°C. This parameter is determined in the case of PVOH by its degree of hydrolysis.

For certain applications or uses, polymers soluble in aqueous environments at temperatures as low as 5°C are also desirable.

In order to ensure that the polymer such as PVOH or cellulose ether is capable of being injection moulded, it is usual to incorporate components such as plasticizers and mould release agents in an amount of up to, for example, 15 wt% of the composition. Suitable plasticizers are, for example, pentaerthyritol such as depentaerythritol, sorbitol, mannitol, glycerine and glycols such as glycerol, ethylene glycol and polyethylene glycol.

Solids such as talc, stearic acid, magnesium stearate, silicon dioxide, zinc stearate, and colloidal silica may also be used. A preferred PVOH which is already in a form suitable for injection moulding is sold in the form of granules under the name CP1210T05 by Soltec Developpement SA of Paris, France.

The PVOH may be moulded at temperatures of, for example, from 180-220°C, depending upon the formulation selected and the melt flow index required. It can be moulded into containers, capsule bodies, caps, receptacles and closures of the appropriate hardness, texture and solubility characteristics.

One of the great practical problems of current hard gelatine capsules is their ability to hold a static electrical charge. Such capsules in production rapidly pick up a high static charge which has the effect of making them not only stick to each other and to all other non-polar surfaces but also making them attract particles of foreign material from their surroundings. It also means that that the capsules are hard to fill, and that their surfaces must be treated immediately prior to printing. This phenomenon is common to some mouldable polymers, but not to PVOH, which is not only soluble, ingestible, mouldable and weldable, but in addition will not support a static charge capable of causing the problems described above. So, yet another consequence of using an injection-moulding method is that the mouldable material may be chosen having regard to its ability to pick up and retain a static charge - or may include one or more additional substances that has some effect on the way the capsule behaves in this respect. Thus the container may be made from materials which do not hold a static charge, such as PVOH or a cellulose ether.

One aspect of the present invention is a container comprising a self-supporting receptacle part and a closure part, the receptacle part and the closure part together enclosing a fabric care, surface care or dishwashing composition, the receptacle part being formed of a water-soluble polymer, and the closure part being formed of a water-soluble polymer, wherein in use, the closure part dissolves before the receptacle part.

Preferably the capsule is a washing capsule enclosing a washing composition.

In many aspects of the present invention, the water-soluble polymer is not limited to PVOH or a cellulose ether. Other water-soluble compounds may be used, such as polyglycolides, gelatine, polylactides and polylactide-polyglycolide copolymers. These components may also, if necessary, contain components such as plasticizers and mould release agents, such as those described above. All of the polymer compositions, including the PVOH and cellulose ether, may also include other components such as colouring agents and components which modify their properties.

In all aspects and embodiments of the present invention, the container generally comprises a receptacle part which holds the composition and a closure part, which may simply close the receptacle part or may itself have at least some receptacle function. The receptacle part preferably has side walls which terminate at their upper end in an outward flange in which the closure part is sealingly secured, especially if the closure part is in the form of a film. The securement may be by means of an adhesive but is preferably achieved by means of a seal, between the flange and the closure part. Heat sealing may be used or other methods such as infra-red, radio frequency, ultrasonic, laser, solvent, vibration or spin welding. An adhesive such as an aqueous solution of PVOH or a cellulose ether may also be used. The seal is desirably also water-soluble.

The closure part may itself be injection moulded or blow moulded. Preferably, however, it is a plastics film secured over the receptacle part. The film may, for example, comprise PVOH or a cellulose ether such as HPMC or another water-soluble polymer.

The container walls have thicknesses such that the containers are rigid. For example, the outside walls and any inside walls which have been injection moulded independently have a thickness of greater than 100µm, for example greater than 150µm or greater than 200µm, 300µm, or 500µm, 750µm or 1mm. Preferably, the closure part is of a thinner material than the receptacle part. Thus, typically, the closure part is of thickness in the range 10 to 200 µm, preferably 50 to 100 µm, and the wall thickness of the receptacle part is in the range 300 to 1500 µm, preferably 500 to 1000 µm. The closure part may, however, also have a wall thickness of 300 to 1500 µm, such as 500 to 1000 µm.

Preferably, the closure part dissolves in water (at least to the extent of allowing the washing composition in the receptacle part to be dissolved by the water; and preferably completely) at 40°C in less than 5 minutes, preferably in less than 2 minutes.

The receptacle part and the closure part could be of the same thickness or different thicknesses. The closure part may, for example, be of higher solubility than the receptacle part, in order to dissolve more quickly.

Preferably, the washing capsule is generally cuboid in its external shape, with the top wall being formed by the closure part, and with the side walls and base wall being formed by the receptacle part.

Preferably, a washing capsule of the invention is manufactured by forming an array of receptacle parts, each receptacle part being joined to adjacent receptacle parts, and being separable from them by a snap or tear action. The array is preferably one which has columns and rows of the receptacle parts. The receptacle parts may be separated by frangible webs of the water-soluble polymer such as PVOH or a cellulose ether.

Alternatively, the receptacle parts may be manufactured with the aforementioned flanges, such that they are separated from each other by a line of weakness. For example the material may be thinner, and so able to be broken or torn readily. The thinness may be a result of the moulding process or, preferably, of a later scoring step.

In the manufacturing method, the array, formed by injection moulding, is fed to a filling zone, and all the receptacle parts are charged with the washing composition. A sheet of a water-soluble polymer such as PVOH or a cellulose ether may then be secured over the top of the array, to form the closure parts for all the receptacle parts of the array. The array may then be split up into the individual washing capsules, prior to packaging, or it may be left as an array, for packaging, to be split by the user. Preferably, it is left as an array, for the user to break or tear off the individual washing capsules. Preferably, the array has a line of symmetry extending between capsules, and the two halves of the array are folded together, about that line of symmetry, so that closure parts are in face-to-face contact. This helps to protect the closure parts from any damage, between factory and user. It will be appreciated that the closure parts are more prone to damage than the receptacle parts. Alternatively two identical arrays of washing capsules may be placed together with their closure parts in face-to-face contact, for packaging.

In some embodiments of the invention the container, capsule or receptacle part may define a single compartment. In other embodiments of the invention the container, capsule or receptacle part may define two or more compartments, which contain different products useful in a washing process. In such a situation a dividing wall or walls of the compartments preferably terminate at the top of the container, capsule or receptacle part i.e. in the same plane as the top edges of the side walls, so that when the receptacle part is closed by the closure part the contents of the compartments cannot mix. The container, capsule or receptacle part may be provided with an upstand, preferably spaced from the side walls thereof, and preferably of generally cylindrical shape. If wished, the remaining volume of the container, capsule or receptacle part can be divided into two or more parts by means of walls extending between the upstand and the side walls.

The container, capsule, receptacle part or closure may be formed with an opening, for example a depression, formed in the side wall or the base wall, and preferably being open in the outward direction. That is to say, it preferably does not form part of the main volume defined by the container, capsule, receptacle part or closure. Preferably the opening is adapted to receive, in a press-fit manner, a solid block (for example a tablet) of a composition, for example a material useful in a washing process.

Preferably, the closure part is of a transparent or translucent material, so that the contents of the washing capsule can be seen.

Preferably, the container, capsule or receptacle part is of a transparent or translucent material, so that the contents of the washing capsule can be seen.

The washing composition within the container, capsule or receptacle part, or within a compartment thereof, need not be uniform. For example during manufacture it could be fed first with a settable agent, for example a gel, useful in a washing process, and then with a different material. The first material could dissolve slowly in the washing process so as to deliver its charge over a long period within the washing process. This might be useful, for example, to provide immediate, delayed or sustained delivery of a softening agent in a clothes washing container, capsule or a receptacle part.

The container, or capsule may, for example, be in at least two parts (a body part and a cap part) which fit tightly, and preferably sealingly and inseparably, together to form a compartment in which is stored the ingredient to be achieved. In one example, the container or capsule may have three parts - a body such as a receptacle, a first cap, and then a second cap to fit over the closed end of either the body or the first cap, so as to result in a capsule with two separate compartments. Where there are three such parts (or more; four parts - a body and three caps - make three compartments, and so on), then naturally the ingredients in each compartment may be the same or they may be different.

In all embodiments of the present invention one compartment may contain, for example, a liquid or solid component (such as a powder, granules or a compressed or gelled tablet) and another may contain a different liquid or solid component (such as a powder, granules or a compressed or gelled tablet). Alternatively, more than one component may be present in one or more compartments. For example a compartment may contain a solid component, for example in the form of a ball or pill (such as a powder, granules or a compressed or gelled tablet), and a liquid component.

By using container, receptacle or capsule cap/body parts of different thicknesses, or of different polymers, or both, such as PVOH polymers with different degrees of hydrolysis, this invention enables enhanced control over the release of different ingredients at different times or in different positions within broad scope of the aqueous destination.

The capsular container can be of any size or shape. It is, for example, conveniently of the standard capsule shape - an elongate tubular package with closed, rounded ends. Moreover, although it is possible to have the several parts of much the same sizes, it is usual that there will be a long body with a shorter cap (the cap may be half or a quarter the length of the body). Typically, a capsular container has an overall closed length of 4 to 10 cm, such as 4 to 6 cm, and an external diameter of 2 to 4 cm. However, it should be understood that there is no theoretical limitation, in either size or shape, and what is suitable will normally be decided upon the basis of the "dose" of the container's contents, the size of any aperture the container may have to pass through, and the available means of delivery.

The capsular container may be in at least two parts (a body or receptacle part and a cap part) which fit tightly, and preferably sealingly and inseparably, together. The actual joining of the parts can be carried out in any convenient way, but advantage can be taken of the very nature of the capsule material - that fact that it is one that can be injection-moulded (it is a thermoplastic). Thus, the preferred joining method is welding, for example either heat welding, by melting the parts when they are in contact, and allowing them to "run" into each other and then cool and solidify to become an integral device, or solvent welding, where much the same effect is achieved by partially dissolving the adjacent portions of the capsule and letting them again run into each other and then solidify to form a whole. Heat welding is much the preferred way, although any of the sealing techniques described herein may be used.

Indeed, in one of its several aspects the invention specifically provides an injection-moulded capsular contained having a cap portion and a body portion which, after filling, are welded together into a single indivisible unit (so sealing in and preventing subsequent access to the contents, and thus ensuring containment of the contents, whether solid, powder, granular, liquid, gel or suspension presentations).

The invention provides a capsule - that is to say, a small container for the relevant ingredients, which container is in at least two parts (a body part and a cap part) which fit tightly, and preferably sealingly and inseparably, together to form a compartment in which is stored the ingredient to be delivered. As an alternative, the capsule may have three parts - a body, a first cap, and then a second cap to fit over the closed end of either the body or the first cap, so as to result in a capsule with two separate compartments. And where there are three such parts (or more; four parts - a body and three caps - make three compartments, and so on), then naturally the ingredients in each compartment may be the same or they may be different.

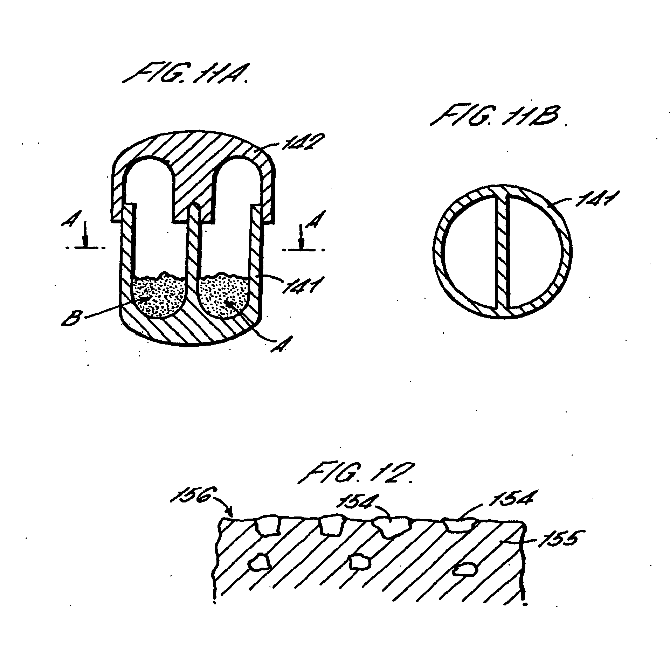

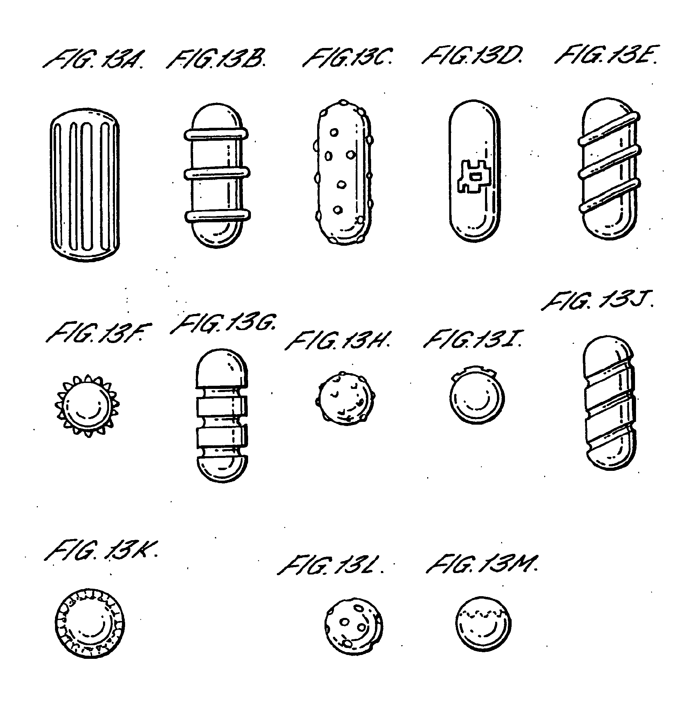

In one example - see Figure 11A in the accompanying Drawings - the capsule may have a body and cap each provided with a central axially-parallel partition, so that the capsule as a whole has two separate compartments.

By using capsule cap/body parts of different thicknesses, or of different polymers, or both, this invention enables enhanced control over the release of different active ingredients at different times or in different positions.

The capsule is of any shape, preferably an elongate tubular package. The ends are advantageously closed, whether rounded or conical. Moreover, although it is possible to have the several parts of much the same sizes, it is usual that there will be a long body with a shorter cap (the cap may be half or a quarter the length of the body) .

Although it is possible to have the several parts of much the same sizes, it is usual that there will be a long body with a shorter cap (the cap may be half or a quarter the length of the body). Typically, a capsular container for applications or uses other than pharmaceutical or nutraceuticals has an overall closed length of 3 to 12 cm, for example 4 to 10 cm and an external diameter of 1 to 5 cm, for example 2 to 4 cm. However, it should be understood that there is no theoretical limitation, in either size or shape, and what is suitable will normally be decided upon the basis of the "dose" of the container's contents, the size of any aperture the container may have to pass through, and the available means of delivery.

In general, PVOH polymers are synthetic materials capable, when appropriately formulated with other adjuvants - such as plasticisers, particularly glycerine (but other glycols and polyglycols may be used depending upon their acceptability for ingestion), and solids such as talc, stearic acid, magnesium stearate, silicon dioxide, zinc stearate, and colloidal silica - of being moulded at temperatures between 180-220°C, depending upon the formulation selected and the melt flow index required, into capsule bodies and caps of the appropriate hardness, texture and solubility characteristics.

PVOH materials, unlike gelatine, can be modified to dissolve at different rates under varying conditions (including the pH of the aqueous medium into which they are introduced). Capsules made from PVOH materials can therefore be formulated to release their contents in any desirable location.

The invention provides a capsule which is in at least two parts (a body part and a cap part) which fit tightly, and preferably sealingly and inseparably, together. The actual joining of the parts can be carried out in any convenient way, but advantage can be taken of the very nature of the capsule material - the fact that it is one that can be injection-moulded (it is a thermoplastic). Thus, the preferred joining method is welding - either heat welding, by melting the parts when they are in contact, and allowing them to "run" into each other and then cool and solidify to become an integral device, or solvent welding, where much the same effect is achieved by partially dissolving the adjacent portions of the capsule and letting them again run into each other and then solidify to form a whole. Heat welding is much the preferred way.

Indeed, in one of its several aspects the invention specifically provides an injection-moulded capsule (suitable for use in the delivery of some active ingredient or device) having a cap portion and a body portion which, after filling, are welded together into a single indivisible unit (so sealing in and preventing subsequent access to the contents, and thus ensuring containment of the contents, whether granular, liquid, gel or suspension presentations).

PVOH materials are particularly suited to thermal welding, a convenient variety of this technique being laser welding, though any suitable method can be used providing it does indeed make a permanent weld with the polymer used to form the capsule. Some other common methods are infra-red (IR), radio frequency (RF), and ultrasonic welding.

Some of these methods may require the addition of other items or processes to ensure their correct operation. For example, RF welding may require the use of a metal (normally aluminium) conductor in content with the capsule surface. Laser welding will normally require the top surface to be transparent to the laser used, and the lower surface to be opaque to it. This can be achieved by avoiding opaque coatings and fillers on the outer surface of the capsule cap and by their application to the outer surface of the capsule body. For example, a circumferential line of a suitable material can be printed around the body at the required joining point to facilitate the weld at that point. As a result of the welding, a circumferential weld situation on a planar cross-section of the capsular container is advantageously obtained.

Of the various methods, the laser weld is preferred as there is no direct contact required, and it can achieve the very high production speeds required.

After placing the intended contents in the capsule body, and putting the cap on the body, the two portions of the capsule can be welded - by means of a laser beam, say - into a single unit which cannot thereafter readily and without leaving visible traces be separated into body and cap in order to gain access to the contents. Accordingly, any attempt to tamper with the contents would be clearly obvious.

The two parts of the capsule that are to be welded together are, for example, made so that the open end of one will pass into the open end of the other with the smallest gap that can be practically achieved to allow easy assembly. Normally, but not necessarily, the capsule is designed with a stop on one or other component so that the entry of one into the other cannot overrun and stops at the same fixed position in every case.

The two halves or shells are in the closed position when the entire periphery of the open end of one is overlapped by the periphery of the open end of the other. The closed capsule is then ready for welding, and this is done by bringing the capsule into close proximity to the welding head. This distance will vary with the method of welding chosen. The welding equipment is operated, and forms a weld between the two layers in contact in the form of a line of weld in a closed loop around the periphery of the capsule. This an be achieved either by having the welding heads in the form of a ring (which may be continuous or made up of a number of discrete heads), or by rotating one or other of the capsule and the head around the other - say, by rolling the capsule past the head. The exact method will depend on the welding technology chosen.

It is also possible to use solvent welding - that is, using a solvent for the chosen injection-mouldable material so as to soften and render flowable the surface layers of the material where the two parts are in contact. In the PVOH case the solvent is conveniently water or an aqueous electrolyte solution (typically containing an alkali metal halide such as lithium chloride as the electrolyte). This technique, however, requires another stage to the welding process, in which the solvent is applied to one of the surfaces to be in contact before the two shells are closed. This method is not preferred, however, as it is likely to be comparatively slow, and the addition of water and solute may well be detrimental to the ingredient(s) or other preparations contained within the capsule.

The weldability of the two parts (body and cap) of the injection-moulded capsule of the invention into a single unit which cannot subsequently be separated into its two parts without visibly destroying the capsule is in contrast to the nature of the known hard gelatine capsule parts, which cannot be so welded. Thus, the integrity of the contents can be protected by the invention's capsule in a way which cannot take place using capsule parts made of gelatine.

Due to the integrity of the welded seal, in all aspects and embodiments the container, receptacle or capsule can be filled with any appropriate powder, liquid, gel, or oil.

The invention provides a capsule, container or receptacle made of a material that can be injection-moulded. The injection-moulding process allows controlled variations in the thickness of the walls and domed ends of either or both halves of the capsule, thereby allowing the release characteristics to be infinitely varied. The use of such moulded capsule shells permits the development of capsule formulations containing controlled-release beads or granules which can be determined where the contents are released so that the system as a whole can be made to deliver its contents at the desired position, rate and period of release irrespective of differing physioco-chemical properties of the contents.

There are many advantages in the production of capsules using injection-moulding as compared with the traditional dip-coating methods, and it is worth setting out a few here.

Dip-coating of gelatine is the traditional method for the production of capsule shells. One of the principal properties of a capsule is the rate at which the shell material dissolves or disperses to release the contained ingredients. Using the dipping process there is only a limited control over the final thickness of the capsule shell. The principal advantage of using the injection-moulding process is that there is much greater versatility over the final component form, for example:-
a) The thickness of the wall sections can be more closely controlled, and hence may be varied inter alia to obtain the appropriate dissolution rate of the capsule.
b) Reduced wall thickness possible with injection-moulded capsule shells will result in increased production rates.
c) The surface form (smoothness) of both inner and outer capsule surfaces can be more closely controlled for moulded as compared with dipping, which latter only allows control of the inner surface form.
d) The degree (tightness) of fit between the two capsule halves can be more closely controlled with moulding.
e) Injection-moulding permits the addition of sectional variation around the rim of either or both of the capsule halves, so that features for final capsule assembly, such as ultrasonic or laser welding, can be included in the basic component design.
f) If both capsule halves are moulded simultaneously, in the same injection-mould tool, the capsule halves can be assembled automatically as a post-moulding operation carried out immediately the tool halves open (with benefits for cleanliness and quality assurance).
g) There are no requirements for further trimming or sizing operations.

In its broadest aspect this invention provides a capsule made of a material that can be injection-moulded. This injection-moulding concept has several unexpected consequences, as does the choice of a polymer of the PVOH type for this purpose. Specifically, an injection-moulded capsule can be moulded in almost any shape that might be useful (as might have been inferred from what has been said above). In particular, it can be given external raised (or lowered) areas.

In another aspect, therefore, the invention provides an injection-moulded capsule (suitable for use in the delivery of some active ingredient or device) having raised portions moulded into its external surface.

Thus the container, capsule, capsular container, receptacle or closure may, for example, have raised portions moulded into its external surface.

The raised portions - for the most part they are referred to hereinafter as "raised", though obviously the effect of a raised part can be achieved by lowering the other parts - can be in the form of short, small pimple-like projections, or they can be ribs that extend wholly or partially either around or along the capsule. The portions may be designed to include or act as markings allowing identification of the capsule and its contents - either visually, by the sighted, or tactilely, by the visually-impaired, or even by a machine or reader. Thus a code can be moulded into the surface so that a filled capsule can be identified at all stages of its life - by the manufacturer for quality assurance and quality control, by a wholesaler or retailer as part of a stock-control system, and by the user before utilisation, particularly those with vision impairment.

The surface of the capsule, container, receptacle or closure needs no pre-treatment prior to printing.

By suitable cutting of the moulds used, any required pattern can be moulded into the surface, either raised or incuse. Both raised and incuse variants bring different properties to the capsule, and the benefits of each are described hereinafter. The complexity of the pattern is limited only by the practical limitations on mould making.

Thinner areas of the walls of different compartments of the capsular container are preferably disposed longitudinally according to the general elongated shape of the capsular container.

The use of an incuse pattern has a number of interesting possibilities. Incuse moulding in a suitable pattern provides a way of converting the capsule from an integral, sealed, container to a perforate container from which the contents of the capsule can readily escape as a solution or suspension (rather like a tea bag, or a metal tea infuser).

Such an incuse pattern design may include a capsule of standard form but with relatively thick walls. Around a suitable section of the capsule is moulded an array of thin-walled incuse panels.

As has been described earlier; PVOH materials can, due to variations in molecular weight and extent of hydrolysis, be selected to dissolve at different speeds and at different temperatures in aqueous conditions. Hence, by varying the thickness and the dissolution characteristics of the injection-moulded capsule materials, the body of the capsule may be designed to dissolve or break up at a chosen rate.

More generally for applications or uses outside of washing, the difference of accessibility time to an aqueous environment from one compartment to another is in the range of 1 minute to 12 hours at the same temperature in the range of 5°C to 95°C.

Another possibility is to mould a capsule in a relatively sparingly-soluble polymer material - such as a high molecular weight PVOH having a high degree of hydrolysis - with a similar array of holes (rather than thin-walled soluble panels), and then in a separate process, after filling and capping, to cover the area containing the holes with a relatively soluble polymer either by spraying or by shrinking or gluing a soluble sleeve thereover.

Another consequence of using an injection-moulding method is that the mouldable material may easily include one or more additional substance that has some effect on the way the capsule behaves in use - for instance, on its surface properties (and specifically on its tackiness, or stickiness), or on its rate of dissolution.

And in still another aspect the invention provides an injection-moulded capsule that is made from an injection-mouldable material that contains one or more particulate solids in order to accelerate the rate of dissolution of the capsule.

The particulate solid incorporated into the injection mix may be a material that is barely affected in a non-acidic medium but dissolves relatively rapidly in an acidic environment, so as to allow the capsule to release its contents. Alternatively, the solid material may be one that is relatively insoluble in an acidic medium but relatively soluble in a neutral environment, so as to allow release of the capsule's contents.

The simple dissolution of the solid in the chosen medium is sufficient to cause a significant acceleration in the capsule break-up, particularly so when a gas is also generated, when the physical agitation caused will result in the virtually immediate release of the contents from the capsule.

Such solids are of course subject to the same limitations of approval and compatibility as before. The solids which can be used for accelerating the rate of dissolution of the capsular container are preferably the bicarbonate and carbonate salts of the alkali and alkaline-earth metals, typically sodium, potassium, magnesium and calcium, all of which salts may liberate carbon dioxide gas for the purpose of generating effervescence.

The solid is very preferably extremely finely divided, typical particle sizes being in the range of 1-25 µm, and preferably 5-10 µm.

Materials that can be utilised to affect the capsule's dissolution rate in a non-acid medium but without being affected by an acid medium are most preferably solid acidic substances with carboxylic or sulphonic acid groups or salts thereof. Substances suitable for this purpose are cinnamic acid, tartaric acid, mandelic acid, fumaric acid, maleic acid, malic acid, pamoic acid, citric acid, and naphthalene disulphonic acid, as free acids or as their alkali or alkaline-earth metal salts, with tartaric acid, citric acid, and cinnamic acid in the form of acids or their alkali metal salts being especially preferred.

One of the great practical problems of current hard gelatine capsules is their ability to hold a static electrical charge. Such capsules in production rapidly pick up a high static charge which has the effect of making them not only stick to each other and to all other non-polar surfaces but also making them attract particles of foreign material from their surroundings. It also means that the capsules are hard to fill, and that their surfaces must be treated immediately prior to printing.

This phenomenon is common to some mouldable polymers, but not to PVOH, which is not only soluble, ingestible, mouldable and weldable, but in addition will not support a static charge capable of causing the problems described above. So, yet another consequence of using an injection-moulding method is that the mouldable material may be chosen having regard to its ability to pick up and retain a static charge - or may include one or more additional substance that has some effect on the way the capsule behaves in this respect.

Another possibility is to mould a capsule, container or receptacle in a relatively sparingly-soluble polymer material - such as a high molecular weight PVOH having a high degree of hydrolysis - with a similar array of holes (rather than thin-walled soluble panels), and then in a separate process, after filling and capping, to cover the area containing the holes with a relatively soluble polymer either by spraying or by shrinking or gluing a soluble sleeve thereover. The relatively-sparingly soluble polymer used in this case could even be an insoluble polymer - provided, of course, that it is injection-moudable.

Another consequence of using an injection-moulding method is that the mouldable material may easily include one or more additional substance that has some effect on the way the capsule behaves in use - for instance, on its rate of dissolution.

Thus, in still another aspect the invention provide a container, for example, relatively-large injection-moulded capsular container, receptacle, capsule or closure that is made from an injection-mouldable material that contains one or more particulate solid in order to accelerate the rate of dissolution of the container. This solid may also be present in the contents of the container, receptacle or capsule.

The simple dissolution of the solid in the chosen medium is sufficient to cause a significant acceleration in the container break-up, particularly so if a gas is also generated, when the physical agitation caused will result in the virtually immediate release of the contents from the container.

The most obvious solids for this purpose are the bicarbonate and carbonate salts of the alkali and alkaline-earth metals, typically sodium, potassium, magnesium and calcium.

The solid is very preferably extremely finely divided, typical particle sizes being the range 1 to 25 µm, and preferably 5 to 10 µm.

Other materials that can be utilised to affect the capsule's dissolution rate are most preferably solid acidic substances with carboxylic or sulphonic acid groups or salts thereof. Substances suitable for this purpose are cinnamic acid, tartaric acid, mandelic acid, fumaric acid, maleic acid, malic acid, pamoic acid, citric acid and naphthalene disulphonic acid, as free acids or as their alkali or alkaline-earth metal salts, with tartaric acid, citric acid, and cinnamic acid in the form of acids or their alkali metal salts being especially preferred.

The container or capsule of the present invention may contain any composition which is intended to be released when the container is placed in an aqueous environment.

Thus it may, for example, contain a fabric care, surface care or dishwashing composition. A fabric care composition is any composition which is used in the field of fabric care, such as in a fabric washing, fabric treating or dyeing process. A surface care composition is any composition which is used in the field of surface care, for example to clear, treat or polish a surface. Suitable surfaces are, for example, household surfaces such as worktops, as well as surfaces of sanitary ware, such as sinks, basins and lavatories. A dishwashing composition is any composition which is used in the field of dishwashing, such as a dishwashing, water-softening or rinse aid composition.

Examples of such compositions are a dishwashing, water-softening, laundry, detergent and rinse-aid compositions. In this case the composition is especially suitable for use in a domestic washing machine such as a clothes washing machine or dishwashing machine. Other examples are disinfectant, antibacterial and antiseptic composition, for example those intended to be diluted with water before use, or a concentrated refill composition, for example for a trigger-type spray used in domestic situations. Such a composition can simply be added to water already held in the spray container.

The container may be used to contain any composition. Desirably the composition has a mass of at least 10 g or 15 g, for example, from 10 g or 15 g to 100 g, especially from 10 g to 15 g to 40 g. For example, a dishwashing composition may weigh from 10 g or 15 g to 20 g, a water-softening composition may weigh from 25 g to 35 g, and a laundry composition may weigh from 10 g to 40 g, 20 g to 40 g or 30 g to 40 g.

The container may also contain, for example, a detergent, pesticide, biocide, deodorant, dye, pigment or water-treatment chemical. It may, for example, deliver detergents or water-treatment chemicals to a washing machine.

For pharmaceutical or nutraceutical applications or uses, the typical mass of the contents of the capsular container is in the range of 10 mg to 15 g, preferably 50 mg to 1 g.

For uses other than pharmaceutical, nutraceutical or washing, the typical mass of the contents of the capsular container is in the range of 1 g to 100 g, preferably 2 g to 50 g.

In general, particularly when used in a domestic environment, the maximum dimension of the container is 5 cm. For example, a cuboid container may have a length of 1 to 5 cm, especially 3.5 to 4.5 cm, a width of 1.5 to 3.5 cm, especially 2 to 3 cm, and a height of 1 to 2 cm, especially 1.25 to 1.75 cm.

The composition contained by the capsule may be, for example, any which is suitable for the designated application, for example a clothes washing or dishwashing application. It may be a powder or a liquid but if a liquid, may be a low water formulation, preferably having a maximum water content of 5 wt%, in order to maintain the integrity of the walls of the capsule or a higher water formulation containing, for example, at least 8 wt% water. The composition may be formulated having regard to the fact that the user will not come into contact with the composition, whether by inhalation or by skin contact. For example, the composition may include an enzyme, without concern about physical contact between the composition containing the enzyme, and the user.

If the container contains an aqueous liquid having a relatively high water content, it may be necessary to take steps to ensure the liquid does not attack the water-soluble polymer if it is soluble in cold water (20°C), or water at a temperature of up to, say, 35°C. Steps may be taken to treat the inside surfaces of the container, for example by coating it with agents such as PVdC (poly(vinylidene chloride))or PTFE (polytetrafluoroethylene), or to adapt the composition to ensure that it does not dissolve the polymer. For example, it has been found that ensuring the composition has a high ionic strength or contains an agent which minimises water loss through the walls of the container will prevent the composition from dissolving the polymer from the inside. This is described in more detail in EP-A-518,689 and WO 97/27743.

The composition held within the container depends, of course, on the intended use of the composition. It may, for example, contain surface active agents such as an anionic, non-ionic, cationic, amphoteric or zwitterionic surface active agent or mixture thereof.

Examples of anionic surfactants are straight-chained or branched alkyl sulfates and alkyl polyalkoxylated sulfates, also known as alkyl ether sulfates. Such surfactants may be produced by the sulfation of higher C₈-C₂₀ fatty alcohols.

Examples of primary alkyl sulfate surfactants are those of formula:

ROSO₃⁻M⁺

wherein R is a linear C₈-C₂₀ hydrocarbyl group and M is a water-solubilising cation. Preferably R is C₁₀-C₁₆ alkyl, for example C₁₂-C₁₄, and M is alkali metal such as lithium, sodium or potassium.

Examples of secondary alkyl sulfate surfactants are those which have the sulfate moiety on a "backbone" of the molecule, for example those of formula:

CH₂(CH₂)ₙ(CHOSO₃⁻M⁺) (CH₂)ₘCH₃

wherein m and n are independently 2 or more, the sum of m+n typically being 6 to 20, for example 9 to 15, and M is a water-solubilising cation such as lithium, sodium or potassium.

Especially preferred secondary alkyl sulfates are the (2,3) alkyl sulfate surfactants of formulae:

CH₂(CH₂)ₓ(CHOSO₃⁻M⁺)CH₃

and

CH₃(CH₂)ₓ(CHOSO₃⁻M⁺)CH₂CH₃

for the 2-sulfate and 3-sulfate, respectively. In these formulae x is at least 4, for example 6 to 20, preferably 10 to 16. M is cation, such as an alkali metal, for example lithium, sodium or potassium.

Examples of alkoxylated alkyl sulfates are ethoxylated alkyl sulfates of the formula:

RO (C₂H₄O)ₙSO₃⁻M⁺

wherein R is a C₈-C₂₀ alkyl group, preferably C₁₀-C₁₈ such as a C₁₂-C₁₆, n is at least 1, for example from 1 to 20, preferably 1 to 15, especially 1 to 6, and M is a salt-forming cation such as lithium, sodium, potassium, ammonium, alkylammonium or alkanolammonium. These compounds can provide especially desirable fabric cleaning performance benefits when used in combination with alkyl sulfates.

The alkyl sulfates and alkyl ether sulfates will generally be used in the form of mixtures comprising varying alkyl chain lengths and, if present, varying degrees of alkoxylation.

Other anionic surfactants which may be employed are salts of fatty acids, for example C₈-C₁₈ fatty acids, especially the sodium, potassium or alkanolammonium salts, and alkyl, for example C₈-C₁₈, benzene sulfonates.

Examples of nonionic surfactants are fatty acid alkoxylates, such as fatty acid ethoxylates, especially those of formula:

R(C₂H₄O)ₙOH

wherein R is a straight or branched C₈-C₁₆ alkyl group, preferably a C₉-C₁₅, for example C₁₀-C₁₄, or C₁₂-C₁₄ alkyl group and n is at least 1, for example from 1 to 16, preferably 2 to 12, more preferably 3 to 10.

The alkoxylated fatty alcohol nonionic surfactant will frequently have a hydrophilic-lipophilic balance (HLB) which ranges from 3 to 17, more preferably from 6 to 15, most preferably from 10 to 15.

Examples of fatty alcohol ethoxylates are those made from alcohols of 12 to 15 carbon atoms and which contain about 7 moles of ethylene oxide. Such materials are commercially marketed under the trademarks Neodol 25-7 and Neodol 23-6.5 by Shell Chemical Company. Other useful Neodols include Neodol 1-5, an ethoxylated fatty alcohol averaging 11 carbon atoms in its alkyl chain with about 5 moles of ethylene oxide; Neodol 23-9, an ethoxylated primary C₁₂-C₁₃ alcohol having about 9 moles of ethylene oxide; and Neodol 91-10, an ethoxylated C₉-C₁₁ primary alcohol having about 10 moles of ethylene oxide.

Alcohol ethoxylates of this type have also been marketed by Shell Chemical Company under the Dobanol trademark. Dobanol 91-5 is an ethoxylated C₉-C₁₁ fatty alcohol with an average of 5 moles ethylene oxide and Dobanol 25-7 is an ethoxylated C₁₂-C₁₅ fatty alcohol with an average of 7 moles of ethylene oxide per mole of fatty alcohol.

Other examples of suitable ethoxylated alcohol nonionic surfactants include Tergitol 15-S-7 and Tergitol 15-S-9, both of which are linear secondary alcohol ethoxylates available from Union Carbide Corporation. Tergitol 15-S-7 is a mixed ethoxylated product of a C₁₁₋C₁₅ linear secondary alkanol with 7 moles of ethylene oxide and Tergitol 15-S-9 is the same but with 9 moles of ethylene oxide.

Other suitable alcohol ethoxylated nonionic surfactants are Neodol 45-11, which is a similar ethylene oxide condensation products of a fatty alcohol having 14-15 carbon atoms and the number of ethylene oxide groups per mole being about 11. Such products are also available from Shell Chemical Company.

Further nonionic surfactants are, for example, C₁₀₋C₁₈ alkyl polyglycosides, such s C₁₂-C₁₆ alkyl polyglycosides, especially the polyglucosides. These are especially useful when high foaming compositions are desired. Further surfactants are polyhydroxy fatty acid amides, such as C₁₀-C₁₈ N-(3-methoxypropyl) glycamides and ethylene oxide-propylene oxide block polymers of the Pluronic type.

Examples of cationic surfactants are those of the quaternary ammonium type.

Examples of amphoteric surfactants are C₁₀-C₁₈ amine oxides and the C₁₂-C₁₈ betaines and sulfobetaines.

The total content of surfactants in the laundry or detergent composition is desirably 60 to 95 wt%, especially 75 to 90 wt%. Desirably an anionic surfactant is present in an amount of 50 to 75 wt%, the nonionic surfactant is present in an amount of 5 to 20 wt%, the cationic surfactant is present in an amount of from 0 to 10 wt% and/or the amphoteric surfactant is present in the amount of from 0 to 10 wt%. These amounts are based on the total solids content of the composition, i.e. excluding the water when present.

Dishwasher compositions usually comprise a detergency builder. Suitable builders are alkali metal or ammonium phosphates, polyphosphates, phosphonates, polyphosphonates, carbonates, bicarbonates, borates, polyhydroxysulfonates, polyacetates, carboxylates such as citrates and other polycarboxylates. The builder is desirably present in an amount of up to 90 wt%, preferably 15 to 90 wt%, more preferably 15 to 75 wt%, relative to the total weight of the composition. Further details of suitable components are given in, for example, EP-A-694,059, EP-A-518,720 and WO 99/06522.

The compositions, particularly when used as laundry washing or dishwashing compositions, may also comprise enzymes, such as protease, lipase, amylase and cellulase enzymes. Such enzymes are commercially available and sold, for example, under the registered trade marks Esperase, Alcalase, Savinase, Termamyl, Lipolase and Celluzyme by Nova Nordisk A/S. Desirably the enzymes are present in the composition in an amount of from 0.5 to 3 wt%, especially 1 to 2 wt%.

The compositions may, if desired, comprise a thickening agent or gelling agent. Suitable thickeners are polyacrylate polymers such as those sold under the trade mark CARBOPOL, or the trade mark ACUSOL by Rohm and Hass Company. Other suitable thickeners are xanthan gums. The thickener, if present, is generally present in an amount of from 0.2 to 4 wt%, especially 0.2 to 2 wt%.

The compositions can also optionally comprise one or more additional ingredients. These include conventional detergent composition components such as further surfactants, bleaches, bleach enhancing agents, builders, suds boosters or suds suppressors, anti-tarnish and anticorrosion agents, organic solvents, co-solvents, phase stabilisers, emulsifying agents, preservatives, soil suspending agents, soil release agents, germicides, phosphates such as sodium tripolyphosphate or potassium tripolyphosphate, pH adjusting agents or buffers, non-builder alkalinity sources, chelating agents, clays such as smectite clays, enzyme stabilizers, anti-limescale agents, colourants, dyes, hydrotropes, dye transfer inhibiting agents, brighteners, and perfumes. If used, such optional ingredients will generally constitute no more than 10 wt%, for example from 1 to 6 wt%, the total weight of the compositions.

The builders counteract the effects of calcium, or other ion, water hardness encountered during laundering or bleaching use of the compositions herein. Examples of such materials are citrate, succinate, malonate, carboxymethyl succinate, carboxylate, polycarboxylate and polyacetyl carboxylate salts, for example with alkali metal or alkaline earth metal cations, or the corresponding free acids. Specific examples are sodium, potassium and lithium salts of oxydisuccinic acid, mellitic acid, benzene polycarboxylic acids, C₁₀-C₂₂ fatty acids and citric acid. Other examples are organic phosphonate type sequestering agents such as those sold by Monsanto under the trade mark Dequest and alkylhydroxy phosphonates. Citrate salts and C₁₂-C₁₈ fatty acid soaps are preferred.

Other suitable builders are polymers and copolymers known to have builder properties. For example, such materials include appropriate polyacrylic acid, polymaleic acid, and polyacrylic/polymaleic and copolymers and their salts, such as those sold by BASF under the trade mark Sokalan.

The builders generally constitute from 0 to 3 wt%, more preferably from 0.1 to 1 wt%, by weight of the compositions.

Compositions which comprise an enzyme may optionally contain materials which maintain the stability of the enzyme. Such enzyme stabilizers include, for example, polyols such as propylene glycol, boric acid and borax. Combinations of these enzyme stabilizers may also be employed. If utilized, the enzyme stabilizers generally constitute from 0.1 to 1 wt% of the compositions.

The compositions may optionally comprise materials which serve as phase stabilizers and/or co-solvents. Example are C₁-C₃ alcohols or diols such as methanol, ethanol, propanol and 1,2-propanediol. C₁-C₃ alkanolamines such as mono-, di- and triethanolamines and monoisopropanolamine can also be used, by themselves or in combination with the alcohols.

If the composition is in liquid form, it may be anhydrous, or, for example, contain up to 5 wt% water. Aqueous compositions generally contain greater than 8 wt% water based on the weight of the aqueous composition. Desirably the aqueous compositions contain more than 10 wt%, 15 wt%, 20 wt%, 25 wt% or 30 wt% water, but desirably less than 80 wt% water, more desirably less than 70 wt%, 60 wt%, 50 wt% or 40 wt% water. They may, for example, contain from 30 to 65 wt% water.

The compositions may optionally comprise components which adjust or maintain the pH of the compositions at optimum levels. Examples of pH adjusting agents are NaOH and citric acid. The pH may be from, for example, 1 to 13, such as 8 to 11 depending on the nature of the composition. For example, a dishwashing composition desirably has a pH of 8 to 11, a laundry composition desirably has a pH of 7 to 9, and a water-softening composition desirably has a pH of 7 to 9.

The composition, such as a washing composition within the container, capsule or receptacle part, or within a compartment thereof if there is more than one compartment, need not be uniform. For example during manufacture it could be fed first with a settable agent, for example a gel, useful in a washing process, and then with a different material. The first material could dissolve slowly in the washing process so as to deliver its charge over a long period within the washing process. This might be useful, for example, to provide delayed or sustained delivery of a softening agent in a clothes washing capsule.

The composition, such as a washing composition may, especially for dishwashing or laundry, include a tablet. Preferably a tablet contains a material useful in a washing process and is formulated to provide slow release of that material during a washing process and/or delayed release thereof. Delayed release may be achieved by providing the tablet with a coating which is slow to dissolve during the washing process. Alternatively the tablet may provide a quick release of components required early in the wash, for example water-softening components and/or enzymes. The tablet may, for example, comprise a disrupting agent, such as one which effervesces when in contact with water such as a combination of citric acid and an alkali metal carbonate or bicarbonate.

A tablet may be provided in the main volume of the receptacle part or may be provided in an outwardly facing opening or depression, as previously described.

When a washing capsule of the invention has a tablet retained in an outwardly facing opening or depression the tablet is preferably one which will not transfer any washing composition to the hands of a user. For example, it may be coated with a soluble polymeric material. As mentioned above, this may also be desirable for delayed release of its charge. If it is desired that the tablet dissolves quickly it may, for example, comprise a disrupting agent such as an effervescing agent.

In accordance with a further aspect of the invention there is provided a method of ware washing, comprising use of a container, receptacle or washing capsule as described and defined above, the method entailing introducing the container, receptacle or washing capsule into a ware washing machine such as a laundry washing machine or dishwashing machine, prior to commencement of the washing process, the container, receptacle or washing capsule being entirely consumed during the washing process.

The invention also provides a capsule - that is to say, a container for the relevant ingredients, which container is in at least two parts (a body part and a cap part) which fit tightly, and preferably sealingly and inseparably, together to form a compartment in which is stored the ingredient to be delivered. In one example - see Figure 11A in the accompanying Drawings - the capsule may have a body and cap each provided with a central axially-parallel partition, so that the capsule as a whole has two separate compartments. In another example the capsule may have three parts - a body, a first cap, and then a second cap to fit over the closed end of either the body or the first cap, so as again to result in a capsule with two separate compartments. And where there are two or three such parts (or more; four parts - a body and three caps - make three compartments, and so on), then naturally the ingredients in each compartment may be the same or they may be different.

The capsule of the invention is one that dissolves in the destined aqueous medium to release its contents therein. The term "dissolve" is used herein in a fairly general sense, to indicate that the capsule crumbles, decomposes, disintegrates or disperses; it need not actually dissolve, although in most cases it will.

The invention will now be further described, by way of example, with reference to the accompanying drawings in which.
Fig. 1 is a perspective view, generally from above, of an array of receptacle parts;
Fig. 2 is a perspective view, generally from above, of an alternative array of receptacle parts;
Fig. 3 is a perspective view of some of the parts shown in Fig. 2, but looking generally from underneath;
Fig. 4 is a perspective view, generally from above, of a third embodiment of receptacle part;
Fig. 5 is a perspective view, generally from above, of the Fig. 4 embodiment, but filled with washing composition and closed over by a closure part, to form a washing capsule of the invention;
Fig. 6 is a perspective view from above of a fourth embodiment of receptacle part; and
Fig. 7 is a perspective view from below of receptacle parts of the type shown in Fig. 6.
Fig. 8A & B show longitudinal cross-sections of a capsular container of the invention in its open and closed states respectively;
Fig. 9 shows the closed capsular container of Fig. 8B but in see-through perspective;
Fig. 10A & B show longitudinal cross-sections of two- and three-compartment capsular containers of the invention;
Fig. 11A & B show respectively longitudinal and transverse cross-sections of another two-compartment capsular container of the invention;
Fig. 12 shows a section through the wall of a solid-filled polymer capsule of the invention;
Fig. 13A-M show various different forms of moulding on and in the surface of capsular containers of the invention.

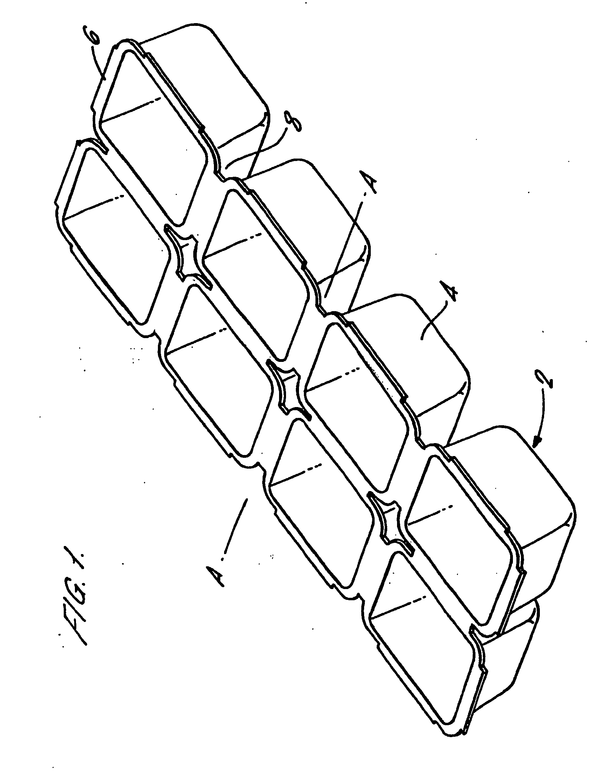

Fig. 1 shows an array of eight receptacle parts 2, arranged as two columns and four rows. Each receptacle part has a flat base wall without indentations or recesses and four uprights side walls 4, and has no top wall. Thus, each receptacle part is upwardly open. Around its opening, at the top of the side walls 4, is an outwardly-directed flange 6, which extends around the entire opening. The receptacle parts are joined to adjacent receptacle parts by webs 8 between the flanges 6. The flanges 6 of all of the receptacle parts lie in one plane. The base walls of all of the receptacle parts also lie in one place, parallel to the plane in which the flanges lie.

The array shown in the drawing is made by injection moulding. The thermoplastic polymer employed in this embodiment is polyvinyl alcohol, and is translucent. The wall thickness is about 0.7 mm. The resulting moulded array is self-supporting.

After injection moulding score lines may be cut into the webs 8 between the flanges, to aid the breaking apart of the washing capsules, for use.

The moulded array is fed to a filling zone where the receptacle parts are simultaneously filled via eight nozzles, with a dishwashing composition. The dishwashing composition could be a powder, gel or paste or could be a liquid formulation. If it is a liquid it may be a liquid formulation of relatively low water content, for example, 2 to 5 wt%, given the properties of the polymer. Alternatively the water content may be higher, for example up to 60 wt% or even 80 wt%, so long as the PVOH is not attacked by the composition. Such steps are described above. A translucent cover film is then laid over the array and heat sealed against the flanges 6, so that each receptacle part has, over it, a closure part. The closure part is also of polyvinyl alcohol, but is much thinner, about 80 µm in this embodiment.

Although the film which constitutes the closure parts is tough it will be appreciated that it is generally less robust than the receptacle parts. In this case, before packaging the product, the capsules may be put into face-to-face contact. An array of washing capsules identical to that of the drawing may be placed in face-to-face contact with it. Alternatively, and conveniently, the array shown in the drawing may be folded about line A-A shown in Fig. 1.

The drawing illustrates the invention but in practice an array of receptacle parts is likely to be considerably larger. Nevertheless, the manufacturing method would be as described.

In use, a user will simply break off a washing capsule from the array, and put it in the dishwashing machine. During the washing process the entire washing capsule will dissolve. The first part to dissolve will generally be the closure part. This may happen very quickly once the washing process starts and the washing composition will immediately be delivered. The receptacle part will generally dissolve more slowly but it will have dissolved entirely by the end of the washing process.

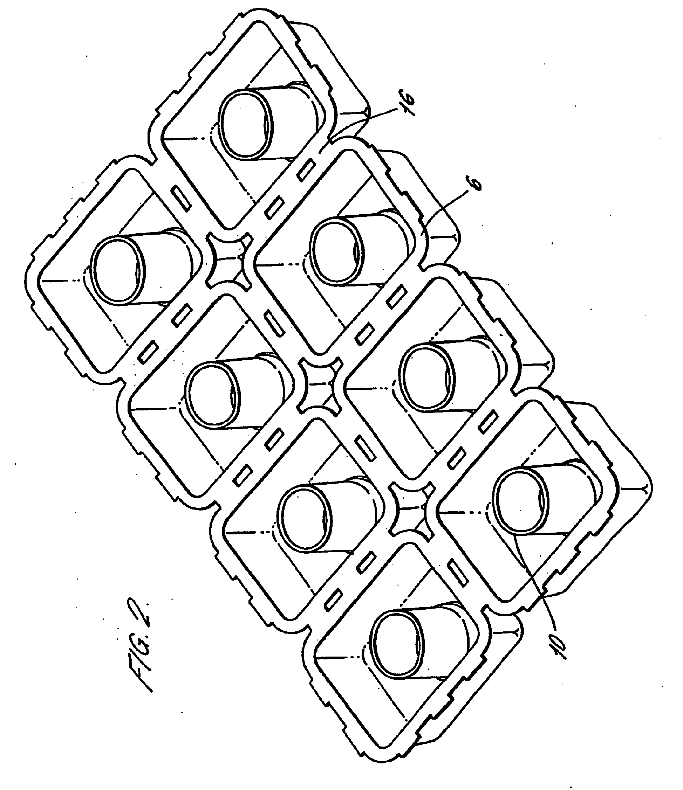

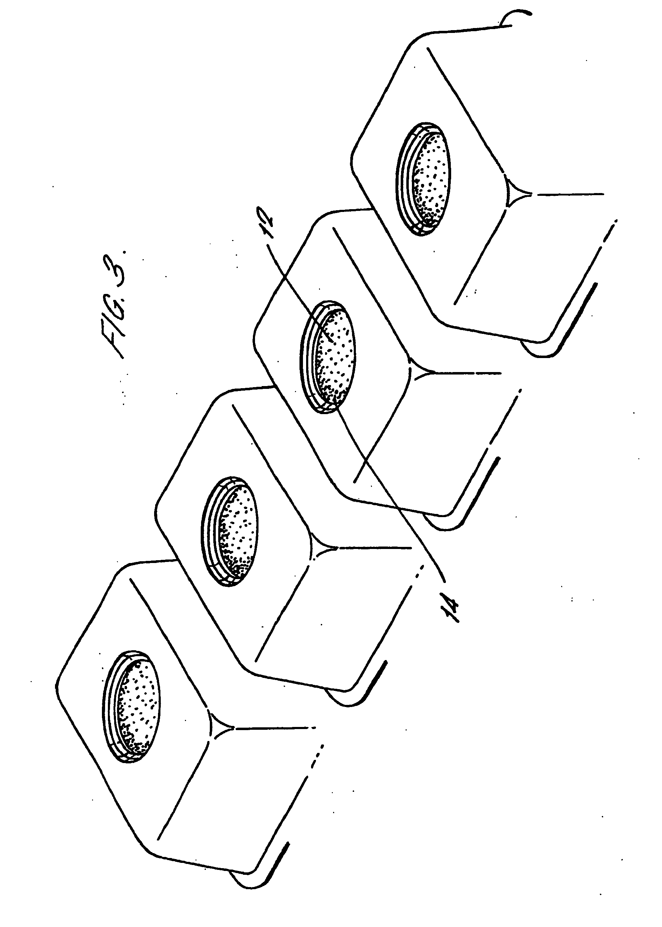

Figs. 2 and 3 show an alternative embodiment of the receptacle parts. The receptacle parts shown in Figs. 2 and 3 are of similar shape and size to those shown in Fig. 1, but have, within the main chamber defined by the base wall and side walls of each receptacle part, a generally cylindrical upstand 10, in a central position. Each upstand is open at its upper end, and its upper end is in the same plane as the flange 6.

As shown in Fig. 3, each receptacle part also has a depression 12 at a central position in its base wall. The depression is relatively shallow, and it is aligned with the upstand 10 carried by the base wall on its other side. Each depression contains within it a tablet 14. Each tablet contains a washing composition or a material which forms part of a washing composition, but is formulated for quick release, slow release and/or delayed release. For slow release it may be a tablet which dissolves over an extended period. For delayed release it may be a table coated with a polymeric coating which is slow to dissolve, so that it releases its charge in the middle or towards the end of a washing cycle.

Another difference between the embodiment of Fig. 2 and that of Fig. 1 is that in the Fig. 2 embodiment there is a plurality of breakable webs 16 of polymeric material extending between the flanges of adjacent receptacle parts.

The array shown in Figs. 2 and 3 is again made by injection moulding, using HPMC polymer having a wall thickness of about 0.8 mm, although PVOH, for example, may also be used. Tablets 14 are press-fitted into the depressions 12 in the undersides of the base walls. The array is then inverted for filling. The upstands 10 are filled with one material, and the remaining volumes, between the upstands and the side walls of the respective receptacle parts, are filled with another material. A cover film is then laid over the array and heat sealed against the flanges 6 and against the ends of the upstands 10, so that each receptacle part has, over it, a closure part. The closure part is of HPMC, about 70 microns thick. Again, PVOH may, for example, also be used.

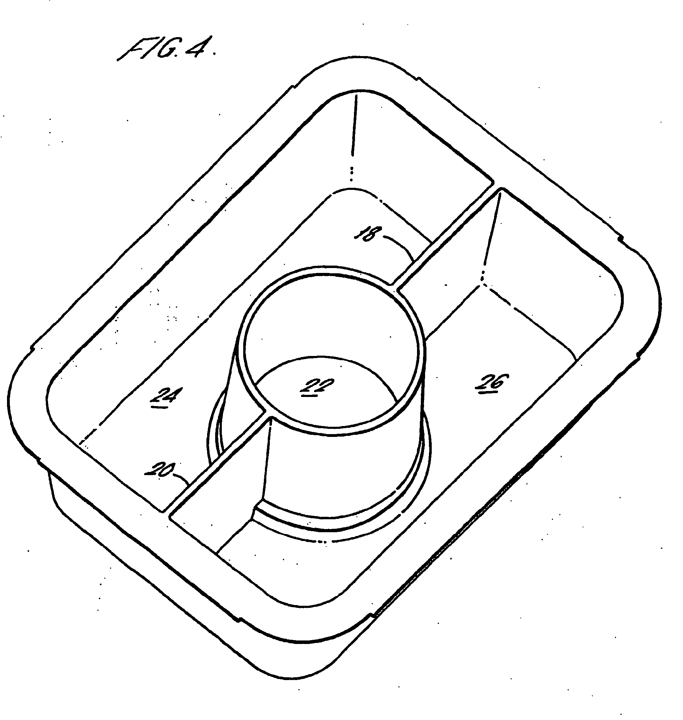

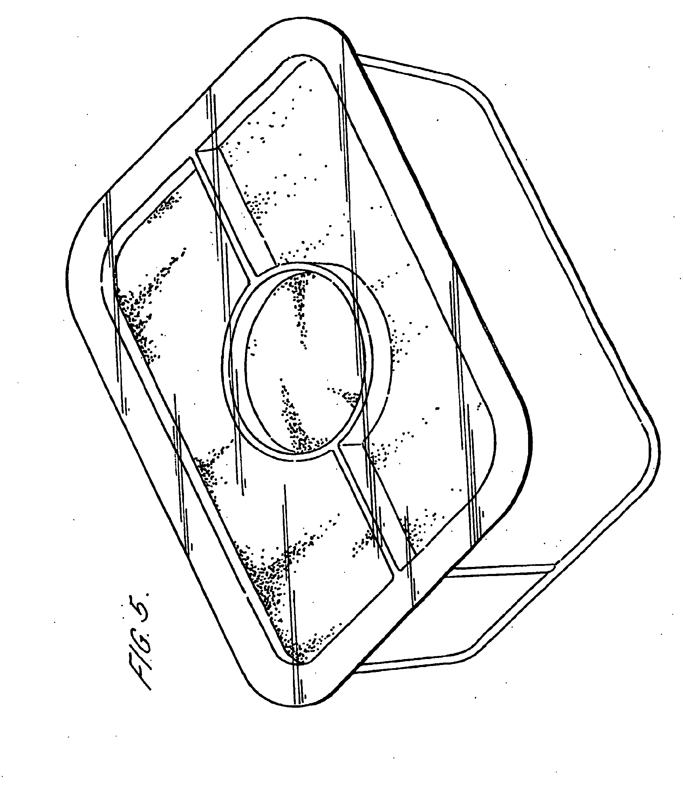

The embodiment shown in Figs. 4 and 5 is similar to that of Figs. 2 and 3 in having an upstand. However the remaining volume of the receptacle part is divided into two by means of walls 18, 20, extending from the upstand in opposed directions, and with each connecting with a respective side wall of the receptacle part. It will be apparent that the receptacle part comprises three main chambers whose contents are released into the washing water once the closure part dissolves. One chamber 22 is defined within the upstand and the other chambers 24, 26 are of identical size to each other and are defined between the upstand and the side walls. The underside of the receptacle part may, like the embodiment of Figs. 2 and 3, comprise a central depression into which is pressed a tablet. The receptacle parts are formed, in an array, by injection moulding.

Fig. 5 shows a washing capsule which uses the receptacle part shown in Fig. 4. The receptacle part has been filled with three different materials useful in a dishwashing cycle and a cover film is shown in place.

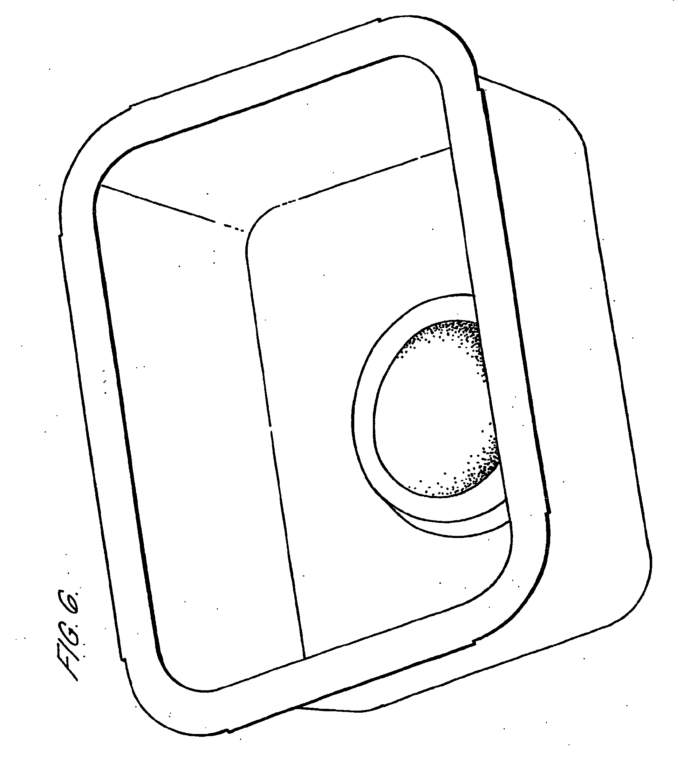

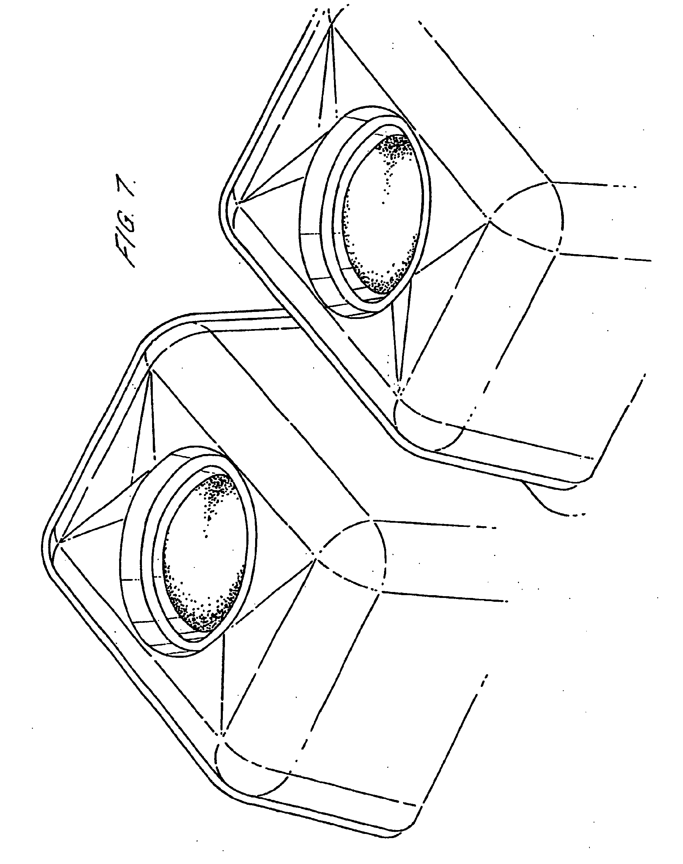

The embodiment of Figs. 6 and 7 is simpler than those of Figs. 2 to 5. The receptacle part shown does not have a central upstand. There is one main volume. However the underside of the base wall is moulded with a depression and into this depression is press-fitted a tablet. In the embodiment of Figs. 6 and 7 the main chamber of the receptacle part can be filled with two or more gels which stay separate, for example, side by side, or one within the other, or in the form of separate stripes. The receptacle parts of Figs. 6 and 7 may be formed, in an array, by vacuum forming.

In the embodiments of Figs. 4 to 7 the materials selected for the receptacle parts and closure parts, and their thicknesses, are as described for the Fig. 1 embodiment.

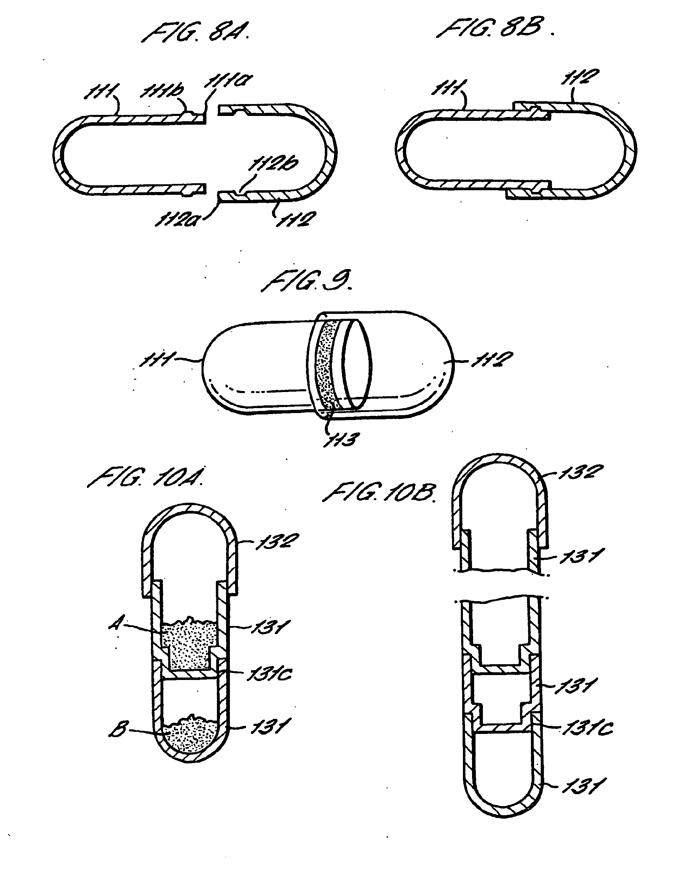

Figure 8 shows a two-part, one compartment capsular container of the invention in its open and its closed form.

The body (111) and cap (112) are to be welded together and are made so that the open end (111a) of one will pass into the open end (112a) of the other with the smallest gap that can be practically achieved to allow easy assembly. There is a "stop" - a ridge (111b) running all round outside of the body 111 that cooperates with a groove (112b) running all round the inside of the cap 112 - so that the entry of one into the other cannot overrun, and stops at the same fixed position in every case.

When the two halves or shells 111, 112 are in the closed position (as in Figure 8B), with the entire periphery of the open end 111a of the body 111 overlapped by the periphery of the open end 112a of the cap 112, the capsular container is ready for welding. The welding equipment (not shown) forms a weld line (113) between the two layers all round the periphery of the container.

Figures 10 and 11 show different sorts of multicompartment capsular container according to the invention.

In Figure 10 the container is made in two or more parts (three in Figure 10A, four are shown in Figure 10B, but there could be more) - in each case there is a single cap portion (132) and a plurality of body portions (as 131). The outer of the body portions 131 is much the same as an "ordinary" body portion (as in Figure 8), but each inner one is shaped at its "outer" end (131c) so that it will fit tightly inside the open mouth of the next body portion, much like in Figure 8 the body 111 fits inside the cap 112.

As shown (in Figure 10A), when the first (outer) body part 131 has been filled with product A, it may then be closed by the second (inner) body part 131 within it. That second body part 131 may then be filled with product B, the cap 132 placed in position, and the three parts welded together at the same time.

Figure 11 shows a capsular container with body (141) and cap (142) two compartments side-by-side (Figure 11B shows a transverse section on the line A-A in Figure 11A). The two compartments can of course hold different products (A and B).

There is theoretically no limit to the number of separate chambers that can be produced either linearly (as in Figure 10) or side by side within the body portion (as in Figure 11). Of course, limitations will be set by practical problems of manufacture.

In Figure 12 there is shown a section through the wall of a solid-filled polymer capsular container of the invention.

Inert solids in powder form have been added to the polymer formulation prior to moulding. This provides a more rigid shell. It especially provides a more rigid capsule shell with a surface less immediately affected by the aqueous content of the mouth or oesophagus, thereby reducing surface tackiness during the initial swallowing. The capsule surface is to a significant extent made up of the particulate insoluble solid ingredient (as 154); the soluble polymer (155) is partially concealed below the contact surface (156).

Figure 13 etc show various different forms of moulding on the surface of capsular containers of the invention, some in the form of cross-sections.

These are sell-evident, and need little comment. Figure 13A, F, for example, shows a capsular container with longitudinal raised ribs, while Figure 13B shows one with lateral (or circumferential) raised ribs and Figure 13E shows one with helical ribs. Figure 13C, H shows a container with raised pimples, while Figure 13D, I shows one with raised identification coding patterns. Figures 13G, J, K, L and M show variants analogues to some of the others, but with incuse rather than raised portions.

The invention is further explained in the following

### Examples.

### EXAMPLES

### Example 1:

### The manufacture of capsules by injection moulding and laser welding

### The moulding stage

Capsules according to the invention were made by the injection moulding method utilising an Arborg 220D (35 tonne) injection moulding machine. The injection cavities were in a two-impression (cap/body) composite water-cooled stainless-steel mould. The PVOH had a material melt flow index of 10-20 grams per 10 mins (DIN 53735).

Injection temperatures were 175°C, 180°C, 180°C and 185°C in the feed, zone 2 and 3, and Nozzle areas. The first stage injection pressure was 400psi (2.8 mPa), and the hold stage pressure was 270psi (1.9 mPa). The pressure well time was 3 secs in the first stage and 5 secs in the hold stage. Tool temperatures were between ambient and 40°C.

The moulding pressures were just sufficient to fill the cavities on the first pressure stage and then sufficient packing pressure to hold on the second stage. Mould open and close rates were as fast as possible.

As noted, the mould layout was divided into two halves, one half moulding capsule bases and the other half capsule caps. After the mould opening sequence, two robotically controlled loading plates pneumatically picked up each capsule half from each tool face. With identical cavity pitch centres, these loading plates were brought together so that each capsule half was located resulting in the usual temporary location of the pair ready for automatic filling.

### The filling stage

For test purposes the capsules were filled by hand with various test materials (see below).

### The welding stage

The closed capsule is introduced into a transparent tube with an internal diameter not more than 20% greater than the external diameter of the capsule. An array of diodes is located circumferentially around the outside of the tube. As the capsule passes by the diode array, a weld is formed. The velocity of the capsule and the power of the IR emitted by the diode array provide the necessary control over the melting process. The IR emission is either continuous or discontinuous. In the case of discontinuous emission, this is achieved by synchronisation of switching depending on the form of weld required and the sensitivity of the contents of the capsule to the IR.

If the characteristics of the material contained within the capsule are such that they absorb the IR, switching of the laser is necessary such that exposure to the IR is limited to the area of the join. This is effected by means of electrical switching or, in a further embodiment, by a form of optical switching using a lens/prism arrangement. In order to overcome the difficulty of synchronisation, again optical fibre delivery of the IR is used to restrict the area of exposure.

### Example 2:

### The manufacture of capsules using laser welding

In an alternative laser welding stage, the laser or other IR source is arranged to focus on the area of the join. This does not create a full circumferential weld but generates a spot weld. Again, the laser is continuously emitting. By forcing the filled capsules to roll (by mechanical means) whilst exposed to the laser, a full circumferential weld results. Alternatively, an optical fibre is used to deliver the IR to the join.

### Test Results

PVOH capsules made in the manner described in Example 1 above were filled with either sugar or tea leaves. They were designed to have a cap portion that would dissolve sooner than the body, and thus open the capsule progressively.

Similarly, a number of conventional gelatine capsules were also prepared and so filled.

In the Test, a capsule was placed in each Test Subject's mouth (in the buccal cavity), and the Subject was asked to note when he/she became aware of the taste of the contents - thus, when the capsule "opened" - and then when the capsule had completely dissolved.

There were two Test Subjects, and each Test was carried out twelve times (for each filling).

The conventional gelatine capsules opened in 3-4 minutes, and dissolved completely in 5-8 minutes. The sugar-filled PVOH capsules of the invention opened in 8-12 minutes, while the tea-filled ones took longer - 14-18 minutes. Complete dissolution took 30-40 minutes in each case.

## Claims

1. An injection-moulded capsule container of any size or shape for the delivery of a water-destined ingredient selected from a fabric care, dishwashing, water-softening, laundry, rinse aid or antibacterial composition or a refill composition for a trigger-type spray, which container is made of a material that will dissolve in the intended aqueous destination site.

2. An injection-moulded, water-soluble capsule container for the delivery into an aqueous environment of detergents or water-treatment chemicals.

3. A container comprising a self-supporting receptacle part and a closure part, the receptacle part and the closure part together enclosing a fabric care, surface care or dishwashing composition, the receptacle part being formed of a water-soluble polymer, and the closure part being formed of a water-soluble polymer, wherein in use, the closure part dissolves before the receptacle part.

4. A container according to claim 4 wherein the receptacle part is made by injection-moulding.

5. An injection-moulded capsule that is made from an injection-mouldable, water-soluble material that contains one or more particulate solids in order to accelerate the rate of dissolution of the capsule, said capsule containing a fabric care, surface care or dishwashing composition.

6. A container according to claim 1, 2, 3, 4 or 5 wherein the container is made of a water-soluble a poly(vinyl alcohol).

7. An injection-moulded capsule container of any size or shape for the delivery of a water-destined ingredient selected from a fabric care, surface care or dishwashing composition, which container is made of a material that will dissolve in the intended aqueous destination site, said material not being a poly(vinyl alcohol).

8. A method of ware washing comprising use of a container as defined in any one of the preceding claims, the method entailing introducing the container into a ware washing machine prior to commencement of the washing process, the container being entirely consumed during the washing process.
